# EUROPEAN PATENT APPLICATION

(11) **EP 4 632 299 A1**
(43) Date of publication of application: **15.10.2025**
(21) Application number: 24169476.9
(22) Date of filing: 10.04.2024
(51) Int. Cl.: F25D 13/00, F25D 21/04, F25D 17/06, F25D 23/06, F25D 27/00, A61H 33/00, A61H 33/06, A61F 7/00

(54) **COLD CHAMBER**

(71) Applicant: Klafs GmbH, 74523 Schwäbisch Hall (DE)
(72) Inventor: GÄBELE, Markus, 74523 Schwäbisch Hall (DE)
(74) Representative: Grünecker Patent- und Rechtsanwälte PartG mbB

(57) **Abstract**

Cold chamber (1) with a walk-in interior space (2) comprising a wall cladding (3), the wall cladding (3) being arranged in front of a wall (4) of the cold chamber in such a way that the wall cladding and the wall form a cavity (5) , the wall being heatable at least partially.

## Description

The present invention relates to a cold chamber according to the features of independent claim 1.

Cold chambers have been used for many years as part of cryotherapy for medical therapies and to treat athletes. Cold chambers are also becoming increasingly popular in the wellness sector, for example to cool down after a sauna session. Typically, the temperatures generated in the cold chamber are significantly higher than the temperatures of cryotherapy. Slowly cooling down after a sauna session at temperatures between 18°C and -5°C is perceived as very pleasant and gentle, especially compared to cooling down with cold water.

Cold chambers in the wellness sector are therefore designed as walk-in rooms, similar to a sauna, in which people can spend a period of time that they find pleasant. The people using the cold chamber can spend the time sitting, lying down or even standing, whereby the ambience in the room is of great importance for a restful and relaxed cool-down. In addition to the prevailing temperature and humidity, the visual design of the cold chamber room, for example in the form of lighting and pictures, has a significant influence on well-being.

A common way to reduce the perceived temperature without increasing the cooling performance is to increases the humidity in the cold chamber, for example by applying a spray mist.

Backlit walls or wall sections are used to create indirect lighting within the cold room and as a special visual element. The backlit wall element forms a kind of cladding element to the actual wall and must be installed at a certain distance from it to allow space for the lighting. This design forms a cavity between the backlit wall and the actual wall.

The design and arrangement of backlit walls was influenced and limited by the fact that moist air from the cold room gets into the cavity between the backlit wall and the actual wall and leads to unwanted condensation causing the wall to become damp.

The task of the present invention is therefore to avoid the aforementioned problem and to provide a cold chamber that ensures a flexibility and freedom in design of the backlit wall.

The problem is solved by the features of independent claim 1. Accordingly the problem is solved in accordance with the invention in that a cold chamber is provided with a walk-in interior space comprises a wall cladding, the wall cladding being arranged in front of a wall of the cold chamber in such a way that the wall cladding and the wall form a cavity, the wall being heatable at least partially.

According to the invention, the term "wall" is not necessarily limited to side walls, but also includes ceilings and even floors. The same applies to the respective wall cladding, which accordingly extends to floor claddings und ceiling claddings.

Advantageous embodiments of the present invention are the subject matter of the dependent claims.

In a preferred embodiment of the present invention, the distance between the wall cladding and the wall is 2-30 cm. The dimension applied can vary depending on the specific design of the cold chamber and it represents a compromise between the needed space between the wall cladding and the wall and an interior space as large as possible. Advantageously the distance is constant across the height and the width of the wall cladding, meaning that the wall cladding and the wall are parallel to each other. However, it is also conceivable that the wall cladding is inclined to the wall so that the distance is not constant but still in the claimed range.

In a preferred embodiment of the present invention, the interior space is coolable and the air temperature is lower than the air temperature of the environment of the cold chamber, the air temperature of the interior space being below 15°C, preferably below 10°C and particularly preferably below 6°C. The set temperature depends on the respective application and the users' needs. The perceived temperature in the cold chamber depends on both the actual air temperature and the air humidity.

In a particularly preferred embodiment of the present invention, the wall cladding is at least partially light-transmissive. It is conceivable that the wall cladding varies in its light transmission rates across its surface, thereby generating patterns. Alternatively, the wall cladding can be uniformly light-transmissive.

According to a preferred embodiment of the present invention, the wall cladding comprises acrylic glass. It is also possible to form the wall cladding by means of an acrylic glass panel.

In a preferred embodiment of the present invention, the wall cladding is attached to the ceiling by means of a suspension device. It is also possible to attach the wall cladding to one or two side walls or to install it on the floor of the cold chamber. Alternatively, the wall cladding could also be attached directly to the wall in front of which it is arranged according to the present invention.

The suspension device separates the cavity from the interior space. However, the cavity is not sealed so that air can exchange between the interior space and the cavity. Moist air from the interior space therefore also penetrates into the cavity. If the air is supersaturated, the moisture in the cavity can condense on the wall and the wall becomes damp. To prevent mold growth, heating elements are therefore provided to dry the wall and evaporate the moisture if necessary.

In a preferred embodiment of the present invention, the cold chamber comprises lighting means so that the cavity between the wall cladding and the wall can be illuminated. Advantageously, the lighting means is installed on the ceiling of the cold chamber and is covered with a cover element to be invisible from the interior space of the cold chamber. In order to illuminate the entire cavity between the wall cladding and the wall, that is, to backlight the wall cladding, the lighting means must be placed behind the wall cladding viewed from the interior space of the cold chamber.

In a preferred embodiment of the present invention, the wall cladding is arranged and designed in such a way that gas exchange occurs between the cavity and the interior space of the cold chamber. In principle, gas exchange is enabled if the cavity between the wall cladding and the wall is not sealed against the interior space. Another option could be to provide holes in the wall cladding to create a connection between the cavity and the interior space.

In a preferred embodiment of the present invention, the wall cladding is arranged in front of the wall in such a way that a gap is formed between an upper edge of the wall cladding and a ceiling of the cold chamber and/or between a lower edge of the wall cladding and a floor of the cold chamber, so that gas exchange between the interior space and the cavity occurs via the gap. Accordingly, the wall cladding is not as wide and/or not as high as the wall so that the cavity is not closed on all sides facilitating gas exchange.

In a preferred embodiment of the present invention, the wall comprises an electric heating element and the wall being heatable by means of the electric heating element at least partially in the area forming the cavity. The wall does not necessarily have to comprise the electric heater in its entirety. It can be either located at a suitable position on the wall or placed outside the cold chamber. It can be sufficient to integrate heating rods into the wall, spaced across a specific area.

Heating the wall has the significant advantage that the wall is not damped by the high humidity in the cold chamber. Instead, it can be dried regularly or as needed.

In a preferred embodiment of the present invention, the wall comprises pipes through which a heating medium can flow so that the wall is heatable at least partially in an area forming the cavity. The pipes are configured for a liquid or a gas to flow through them. It is certainly conceivable, for example, to pass warm exhaust air from a nearby sauna through the pipes and thereby heat the wall. This procedure is particularly effective during the cold chamber closing time.

In a particularly preferred embodiment of the present invention, the cold chamber has a door through which the users of the cold room can enter and leave the cold chamber. The present invention is particularly suitable where the cold chamber is provided as part of a larger wellness facility that also includes saunas and swimming pools. Due to the regular access of users of the cold chamber who are wet or sweaty, the humidity in the cold chamber repeatedly increases, which can lead to the air becoming oversaturated and no longer being able to absorb the moisture. In a particularly preferred embodiment of the present invention, the cold chamber therefore comprises a cooling element on which the humidity can freeze. This cooling element can, for example, be included in the design of the cold chamber and be designed as a visually appealing snow wall. If the cold chamber is heavily frequented, it is nevertheless possible that local moisture peaks may occur due to turbulence in the interior space air and that the moisture does not freeze on the snow wall but enters the cavity.

In a preferred embodiment of the present invention, the cold chamber comprises a ventilation means that is configured to ventilate the cavity between the wall and the wall cladding. It is conceivable, for example, that the moist air is extracted at one area of the cavity and dry air is supplied at another area. The ventilation process does not necessarily have to be continuous. It may also be sufficient to ventilate the cavity only from time to time, for example during the cold chamber closing time.

In a preferred embodiment of the present invention, the cold chamber comprises a drainage means configured to drain water from the cavity. One possibility is to let the floor in the area of the cavity slope towards an opening so that the water can drain away there.

In the following, one embodiment of the cold chamber according to the invention is explained in more detail with reference to a drawing.

In the drawing:

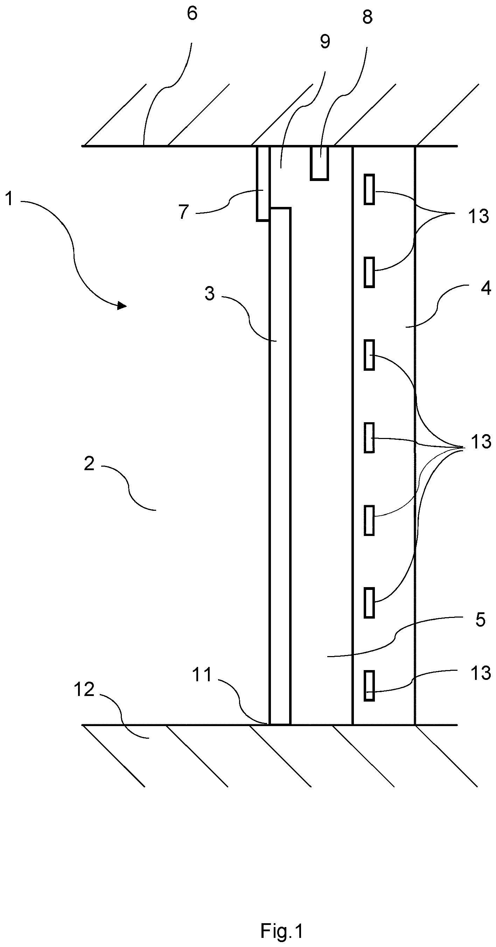

Figure 1: shows a schematic partial side view of a cold chamber according to the invention.

In the following Figures, identical parts are identified by the same reference signs. Insofar as a Figure contains reference signs which are not explicitly referred to in the associated Figure description, reference is made to preceding or subsequent Figure descriptions.

Figure 1 shows a schematic partial view of a cold chamber 1 having a wall 4 cladding 3 arranged in front of a wall 4, the wall 4 being a side wall of the cold chamber 1. It can also be seen that the wall cladding 3 is not as high as the wall 4 so that a gap 9 forms between an upper edge 10 of the wall cladding 3 and the ceiling 6 and that the cavity 5 formed by the wall cladding 3 and the wall 4 is open at the top. The wall cladding 3 is attached to the ceiling 6 of the cold chamber 1 by means of a suspension device 7. Above the wall cladding 3 the suspension device 7 separates the cavity 5, formed by the wall cladding 3 and the wall 4, from the interior space of the cold chamber 1. Additionally the suspension device 7 covers the lighting means 8 that is located on the ceiling 6 behind the wall cladding 3 viewed from the interior space 2. Figure 1 also shows heating elements 13 spaced across the height of the wall 4.

The suspension device 7 separates the cavity 5 from the interior space 2. However, the cavity 5 is not sealed so that air can exchange between the interior space 2 and the cavity 5. Moist air from the interior space 2 therefore also penetrates into the cavity 5. If the air is supersaturated, the moisture in the cavity 5 can condense on the wall 4 and the wall 4 becomes damp. To prevent mold growth, heating elements 13 are therefore provided to dry the wall 4 and evaporate the moisture if necessary.

### List of Reference Signs

- 1: cold chamber
- 2: interior space
- 3: wall cladding
- 4: wall
- 5: cavity
- 6: ceiling
- 7: suspension device
- 8: lighting means
- 9: gap
- 10: upper edge of the wall cladding
- 11: lower edge of the wall cladding
- 12: floor

## Claims

1. Cold chamber (1) with a walk-in interior space (2) comprising a wall cladding (3), the wall cladding (3) being arranged in front of a wall (4) of the cold chamber (1) in such a way that the wall cladding (3) and the wall (4) form a cavity (5), the wall (4) being heatable at least partially.

2. Cold chamber (1) according to claim 1, **characterized in that** the distance between the wall cladding (3) and the wall (4) is 2-30 cm.

3. Cold chamber (1) according to one of claims 1 or 2, **characterized in that** the interior space (2) is coolable and the air temperature is lower than the air temperature of the environment of the cold chamber, the air temperature of the interior space (2) being below 15°C, preferably below 10°C and particularly preferably below 6°C.

4. Cold chamber (1) according to one of claims 1 to 3, **characterized in that** the wall cladding (3) is at least partially light-transmissive.

5. Cold chamber (1) according to one of claims 1 to 4, **characterized in that** the wall cladding (3) comprises acrylic glass.

6. Cold chamber (1) according to one of claims 1 to 5, **characterized in that** the wall cladding (3) is attached to the ceiling (6) of the cold chamber (1) by means of a suspension device (7).

7. Cold chamber (1) according to one of claims 1 to 6, **characterized in that** the cold chamber (1) comprises lighting means (8) so that the cavity (5) between the wall cladding (3) and the wall (4) can be illuminated.

8. Cold chamber (1) according to one of claims 1 to 7, **characterized in that** the wall cladding (3) is arranged and designed in such a way that gas exchange occurs between the cavity (5) and the interior space (2) of the cold chamber (1).

9. Cold chamber (1) according to claim 8, **characterized in that** the wall cladding (3) is arranged in front of the wall (4) in such a way that a gap (9) is formed between an upper edge (10) of the wall cladding (3) and a ceiling (6) of the cold chamber (1) and/or between a lower edge (11) of the wall cladding (3) and a floor (12) of the cold chamber (1), so that gas exchange between the interior space (2) and the cavity (5) occurs via the gap (9).

10. Cold chamber (1) according to one of claims 1 to 9, **characterized in that** the wall (4) comprises an electric heating element (13) and the wall (4) being heatable by means of the electric heating element (13) at least partially in the area forming the cavity (5).

11. Cold chamber (1) according to one of claims 1 to 9, **characterized in that** the wall (4) comprises pipes through which a heating medium can flow so that the wall (4) is heatable at least partially in an area forming the cavity (5).

12. Cold chamber (1) according to one of claims 1 to 11, **characterized in that** the cold chamber (1) comprises a ventilation means that is configured to ventilate the cavity (5) between the wall (4) and the wall cladding (3).

13. Cold chamber (1) according to one of claims 1 to 12, **characterized in that** the cold chamber (1) comprises a drainage means configured to drain water from the cavity (5).
